# EUROPEAN PATENT APPLICATION

(11) **EP 4 169 555 A1**
(43) Date of publication of application: **26.04.2023**
(21) Application number: 22202719.5
(22) Date of filing: 20.10.2022
(51) Int. Cl.: A61M 5/38, A61M 5/165, B01D 19/00

(54) **PRESSURE-ASSISTED AIR ELIMINATION**

(30) Priority: 25.10.2021 US 202163271318 P
(71) Applicant: Fresenius Kabi USA, LLC, Lake Zurich, IL 60047 (US)
(72) Inventor: Powers, Benjamin G., Portsmouth, 03801 (US); Ambrosina, Jesse E., Topsfield, 01983 (US); Scarsella, Michael J., Merrimac, 01860 (US); Gonzalez, Lino A., Parkland, 33076 (US)
(74) Representative: Cohausz & Florack

(57) **Abstract**

A liquid delivery system includes an air elimination assembly disposed in a pathway between a liquid source and a recipient. As its name suggests, the air elimination assembly removes gas from the liquid as it flows between an input port and output port of the air elimination assembly. A magnitude of pressure at the gas output port of the air elimination assembly is controlled to expel gas from the liquid passing from the input port to the output port. The gas expelled from the liquid is outputted from the gas output port. The liquid delivered to the recipient is void of any gases.

## Description

### RELATED APPLICATION

This application claims the benefit of earlier filed United States Provisional Patent Application Serial Number 63/271,318 entitled "PRESSURE-ASSISTED AIR ELIMINATION," (Attorney Docket No. FLU21-01P), filed on October 25, 2021, the entire teachings of which are incorporated herein by this reference.

### BACKGROUND

Air entrained in the liquid path is a major issue in the field of infusion therapy. Air delivered into a patient's vascular system can result in embolism, leading to serious medical complications. To prevent the infusion of air to a patient, infusion pumps typically include bubble sensors, also known as air in line sensors, along the liquid path to sense if bubbles bigger than a given threshold size are present in the line and, if so, trigger an alarm to notify the clinician. Those sensors are typically optical or ultrasonic in nature.

However, while sounding an alarm when air is present prevents the risk of an air embolism, it still induces other problems. The first issue is that the infusion is stopped, resulting in the delay of a potential life-sustaining therapeutic drug. Furthermore, the clinician will typically need to disconnect the liquid path from the patient to purge the air bubble, thus introducing risk of contaminating the connection site and causing an infection in the patient. Still further, because it is difficult to assess what is a safe amount of air to infuse into a patient, thresholds for bubble detectors are typically set relatively low in order to alert the clinician of a potentially hazardous condition.

Thus, a clinician relies on the bubble detector to make the assessment if the size of the bubble is safe to infuse or not. This leads to many of the alarms being "false alarms," which not only result in stoppage of flows, but also contributes to so-called "alarm fatigue" of the clinicians, which has been cited as a major issue in modern hospitals. In other words, there are so many false alarms that a clinician may discontinue to pay attention to them.

### BRIEF DESCRIPTION OF EXAMPLES

This disclosure includes the observation that, while bubble detectors are an important safety element for infusion pumps, a better solution would be to remove air from the liquid path altogether rather than sound an alarm if bubbles are detected.
liquidliquidThe examples as discussed herein prevent or reduce the risks of air embolism, delay of therapy, and alarm fatigue by using negative pressure and a hydrophobic membrane to actively draw air out of the liquid path. For example, certain techniques herein include removal of air entrained in a liquid flow path by use of negative gauge pressure applied on the air side of a hydrophobic (or oleophobic) vent membrane, removing gas from the liquid and in the liquid path. Further examples as discussed herein include multiple configurations ensuring that bubbles are removed prior to delivery of the liquid to a recipient. One example herein includes the use of a pneumatically driven liquid pump to provide the negative pressure applied to an air elimination assembly to draw the air out of the liquid path.

More specifically, a liquid delivery system includes an air elimination assembly disposed at any of one or more locations in a pathway such as between a liquid source and a recipient. As its name suggests, the air elimination assembly removes or reduces presence of gas from the liquid, such as during a condition in which the liquid flows between a liquid input port (a.k.a., inlet port) and liquid output port (a.k.a., outlet port) of the air elimination assembly. The magnitude of pressure at the gas output port of the air elimination assembly is controlled, such as via a controller, to expel gas from the liquid passing from the input port to the output port of the air elimination assembly. The gas expelled from the liquid is safely outputted from the gas output port.

Accordingly, a method herein includes: receiving liquid at an input port of an air elimination assembly; controlling a magnitude of pressure at a gas output port of the air elimination assembly to expel gas from the liquid passing from the input port to the output port, the gas outputted from the gas output port; and outputting the liquid from an output port of the air elimination assembly.

In further examples, the method includes controlling the magnitude of pressure at the gas output port. This may include, via the controller or other suitable entity, setting the pressure at the gas output port to be lower than a magnitude of pressure of the liquid inputted to the input port. If desired, a difference between the magnitude of pressure at the gas output port and the magnitude of pressure of the liquid inputted to the input port is greater than 1 PSI (Pound per Square Inch) although the difference can be any suitable amount.

In yet further examples, the air elimination assembly includes a first membrane between the input port and the gas output port. The first membrane allows passage of gas from the liquid to the gas output port while preventing the liquid from passing through the membrane to the gas output port. Note that the controller can be configured to set the magnitude of the pressure at the gas output port based on attributes of the first membrane.

In further examples, the air elimination assembly includes a first membrane disposed between the input port and the gas output port. A controller sets the magnitude of the pressure at the gas output port based on attributes of the liquid and/or first membrane. The attributes of the liquid used to control the pressure at the gas output port include one or more of: i) a viscosity of the liquid, and ii) a surface tension of the liquid. The attributes of the first membrane used to the control the pressure at the gas output port include one of more of: i) the water-entry pressure of the membrane, ii) the pore size of the membrane, and iii) the material of the membrane.

Further examples herein include, via the controller, monitoring a magnitude of the pressure of the liquid inputted to the input port; deriving a pressure value from the magnitude of the pressure of the liquid inputted to the input port, the derived pressure value lower than a pressure of the liquid at the input port; and setting the pressure at the gas output port to be the derived pressure value.

In still further examples, the liquid delivery system is described herein includes a respective diaphragm pump. The controller controls operation of the diaphragm pump to deliver the liquid through the air elimination assembly to a recipient. Operation of the diaphragm pump includes application of positive air/gas pressure and negative air/gas pressure to a chamber of the diaphragm pump. The controller can be configured to utilize the negative air/gas pressure associated with control of the diaphragm pump (and flow of the liquid) to set the magnitude of pressure at the gas output port of the air elimination assembly.

Yet further examples herein include, via the controller, varying a magnitude of the pressure applied at the gas output port of the air elimination assembly during conveyance of the liquid through the air elimination assembly from the input port to the output port. Controlling the magnitude of pressure at the gas output port (such as with the negative pressure with respect to the pressure of the liquid at the input port) causes the gas in the liquid to pass through a membrane of the air elimination assembly to the gas output port instead of being delivered downstream to the recipient.

In further examples, the air elimination assembly includes: a first membrane through which the gas in the liquid passes to the gas output port and further includes a second membrane through which the liquid passes from the input port to the output port. The second membrane can be configured to prevent passage of the gas from the liquid input port to the output port as well. In one example the second membrane can be configured to be hydrophilic to prevent the passage of gas from the liquid input port to the output port.

The first membrane can be disposed at a higher position above a ground level in the air elimination assembly than the second membrane; a buoyancy of the gas in the air elimination assembly causes at least a portion of the gas in the liquid to flow from a level of the second membrane to a level of the first membrane.

In still further examples, the air elimination assembly includes a chamber disposed between the input port and the output port; the chamber has a cross-section (orthogonal to liquid flow) substantially larger than a cross section (orthogonal to liquid flow) of the input port. The larger cross-sectional area of the chamber reduces the velocity of the liquid flow such that the buoyancy forces on the bubbles overcome the drag forces imparted on the gas bubbles by the liquid flow. In one configuration, the larger cross-sectional area of the chamber and/or reduced velocity of the liquid from the input port to the output port allows the bubbles to float up to the gas outlet port rather than being drawn to the liquid outlet by the liquid flow.

In further examples, the air elimination assembly includes a chamber disposed between the input port and the output port; the chamber includes a tapered pathway extending to the gas output port. In one example, the gas output port is disposed at a higher level than one or more of the input port and/or the output port with respect to a ground level. Buoyancy of the gas in the liquid causes the gas to rise and travel through the tapered pathway to gas output port.

In yet further examples, the air elimination assembly includes multiple gas output ports such as a first gas output port and a second gas output port. The air elimination assembly includes a chamber disposed between the input port and the output port. The chamber can be configured to include a first tapered pathway extending to the first gas output port; the chamber can be configured to include a second tapered pathway extending to the second gas output port. The first tapered pathway is disposed opposite the second tapered pathway. In one example, the input port is disposed between the first tapered pathway and the second tapered pathway; the output port is also disposed between the first tapered pathway and the second tapered pathway.

These and other more specific examples are disclosed in more detail below.

Note that any of the resources as discussed herein can include one or more computerized devices, liquid delivery systems, medical equipment, or the like to carry out and/or support any or all of the method operations disclosed herein. In other words, one or more computerized devices or processors can be programmed and/or configured to operate as explained herein to carry out different examples of the invention.

Yet other examples herein include software programs to perform the steps and operations summarized above and disclosed in detail below. One such example comprises a computer programable product including a non-transitory computer-readable storage medium (i.e., any physical computer readable hardware storage medium) on which software instructions are encoded for subsequent execution. The instructions, when executed in a computerized device (e.g., computer processing hardware) having a processor, program and/or cause the processor to perform the operations disclosed herein. Such arrangements are typically provided as software, code, instructions, firmware, and/or other data (e.g., data structures) arranged or encoded on a non-transitory computer readable storage medium such as an optical medium (e.g., CD-ROM), floppy disk, hard disk, memory stick, etc., or other medium stored in one or more type of memory such as ROM, RAM, PROM, etc., or as an Application Specific Integrated Circuit (ASIC), etc. The software or firmware or other such configurations can be installed onto a computerized device to cause the computerized device to perform the techniques explained herein.

Accordingly, examples herein are directed to a method, system, computer program product, etc., that supports operations as discussed herein.

One example herein includes a computer readable storage medium and/or system having instructions stored thereon. The instructions, when executed by computer processor hardware, cause the computer processor hardware to: control a magnitude of pressure at a gas output port of an air elimination assembly to remove gas from liquid flowing between an input port to an output port of the air elimination assembly, the gas being outputted from the gas output port based on the controlled magnitude of pressure at the gas output port.

The ordering of the operations above has been added for clarity sake. Note that any of the processing steps as discussed herein can be performed in any suitable order.

Other examples of the present disclosure include software programs and/or respective hardware to perform any of the method example steps and operations summarized above and disclosed in detail below.

It is to be understood that the system, method, apparatus, instructions on computer readable storage media, etc., as discussed herein also can be embodied strictly as a software program, firmware, as a hybrid of software, hardware and/or firmware, or as hardware alone such as within a processor, or within an operating system or within a software application.

As discussed herein, techniques herein are well suited for the removal of gas from a liquid delivery system. However, it should be noted that examples herein are not limited to use in such applications and that the techniques discussed herein are well suited for other applications as well.

Additionally, note that although each of the different features, techniques, configurations, etc., herein may be discussed in different places of this disclosure, it is intended, where suitable, that each of the concepts can optionally be executed independently of each other or in combination with each other. Accordingly, the one or more present inventions as described herein can be embodied and viewed in many different ways.

Also, note that this preliminary discussion of examples herein purposefully does not specify every example and/or incrementally novel aspect of the present disclosure or claimed invention(s). Instead, this brief description only presents general examples and corresponding points of novelty over conventional techniques. For additional details and/or possible perspectives (permutations) of the invention(s), the reader is directed to the Detailed Description section and corresponding figures of the present disclosure as further discussed below.

### BRIEF DESCRIPTION OF THE DRAWINGS

FIG. 1 is an example diagram illustrating a liquid delivery system as discussed herein.
FIG. 2 is an example diagram illustrating implementation of an air elimination assembly as discussed herein.
FIG. 3 is an example diagram illustrating implementation of an air elimination assembly as discussed herein.
FIG. 4 is an example diagram illustrating implementation of an air elimination assembly as discussed herein.
FIG. 5 is an example diagram illustrating implementation of an air elimination assembly including a tapered end as discussed herein.
FIG. 6 is an example diagram illustrating implementation of an air elimination assembly including multiple tapered ends as discussed herein.
FIG. 7 is an example diagram illustrating implementation of an air elimination assembly including a valve as discussed herein.
FIG. 8 is an example diagram illustrating a liquid delivery system as discussed herein.
FIG. 9A is an example diagram illustrating implementation of multiple air elimination assemblies at different locations in a liquid pathway of a cassette as discussed herein.
FIG. 9B is an example diagram illustrating implementation of multiple air elimination assemblies at different locations in a liquid pathway of a cassette as discussed herein.
FIG. 10 is an example diagram illustrating implementation of a cassette including one or more air elimination assemblies as discussed herein.
FIG. 11 is an example diagram illustrating a computer architecture in which to execute one or more techniques (operations) as discussed herein.
FIG. 12 is an example diagram illustrating a method as discussed herein.

The foregoing and other objects, features, and advantages of the invention will be apparent from the following more particular description of preferred examples herein, as illustrated in the accompanying drawings in which like reference characters refer to the same parts throughout the different views. The drawings are not necessarily to scale, with emphasis instead being placed upon illustrating the examples, principles, concepts, etc.

### DETAILED DESCRIPTION AND FURTHER SUMMARY OF EXAMPLES

A liquid delivery system includes an air elimination assembly disposed in a pathway between a liquid source and recipient. As its name suggests, the air elimination assembly removes gas such as during conveyance of the liquid along the pathway between an input port and output port of the air elimination assembly. A magnitude of pressure at the gas output port of the air elimination assembly is controlled to expel gas from the liquid passing from the input port to the output port. The gas expelled from the liquid is outputted from the gas output port such that the liquid delivered from the air elimination assembly to the recipient is substantially void of any gases.

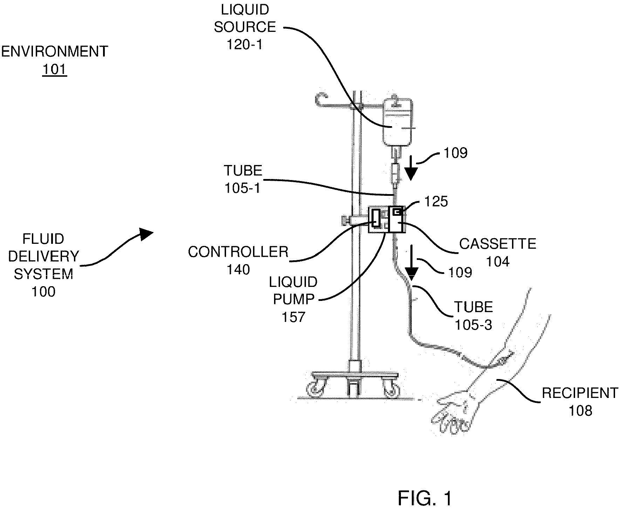

Now, more specifically, FIG. 1 is an example diagram illustrating a liquid delivery system as discussed herein.

FIG. 1 shows an example liquid delivery configuration for infusion of medical liquids. One or more liquids are delivered from a liquid source 120-1 to the recipient 108 (such as human, animal, manufacturing equipment, etc.) through the liquid path including tubing 105-1, cassette 104, tubing 105-3). The liquid flow is regulated by the controller 140, also referred to herein as a "pump."

Note that the liquid pump 157 and corresponding liquid path between liquid source 120-1 and the recipient 108 can be configured to include a cassette 104 that interfaces with the controller 140. During delivery of the liquid 109 such as from source 120-1, air (i.e., any gas) can become entrained (conveyed) in the flow of liquid 109 via one or more means.

For example, air from the liquid source 120 might enter the tubing 105-1 if the source 120-1 is tilted or jostled during delivery. Air (i.e., gas) can also outgas from the liquid 109 itself such as when the liquid 109 warms, one or more chemical reactions occur in the liquid 109, or as the pumping action of the controlled liquid 109 delivery causes pressure fluctuations in the liquid 109.

Any of these mechanisms, and others, can lead to air entering the liquid path and causing either an air-in-line air detection alarm to sound or air delivery to the recipient 108. When air is in the liquid 109 and delivered to the recipient 108 such as a human, this may cause an air embolism to the recipient 108. If the recipient (non-human) is manufacturing equipment, the presence of air in the liquid 109 may cause damage to the equipment itself or damage to the corresponding product being manufactured by the equipment.

As further discussed herein, the cassette 104 can be configured to include one or more air elimination assemblies 125 disposed in the liquid path that draws air out of the liquid 109 to prevent delivery of same to the recipient 108.

Yet further, as further discussed herein, note that a basic configuration of the air elimination assembly 125 includes two primary elements. The first is a vent (such as membrane) that allows air to pass from a first surface to an opposite surface, but prevents the passage of liquid through the vent. This vent could be a hydrophobic membrane with appropriate pore size and material properties to prevent liquid flow but allow air passage. Typically these vents might be made from PTFE (a.k.a., Polytetrafluoroethylene) or PES (a.k.a., Polyethersulfone) polymers and have pore sizes from 0.022 um (i.e., 0.022 micrometers) up to 0.45 um or other suitable values. Alternately the vents might be oleophobic in nature. Note that oleophobic membranes can also be used in the air elimination assembly 125. Such membranes are typically similar material and have a respective pore size similar to hydrophobic membranes, but have additional chemical treatments to also prevent the passage of lower surface energy liquids such as oils or alcohols.

As further discussed herein, the second primary element of the air elimination assembly 125 (such as in the cassette 104) is negative gauge pressure applied to the air side of the vent membrane. In other words, in one example, a magnitude of pressure applied to the air side of the vent membrane (gas output port) is less than a magnitude of pressure in the liquid pathway between the input port and the output port. The negative pressure applied to the gas output port actively draws out any bubbles in the liquid path that come in contact with the vent membrane. Details are discussed with respect to the following figures.

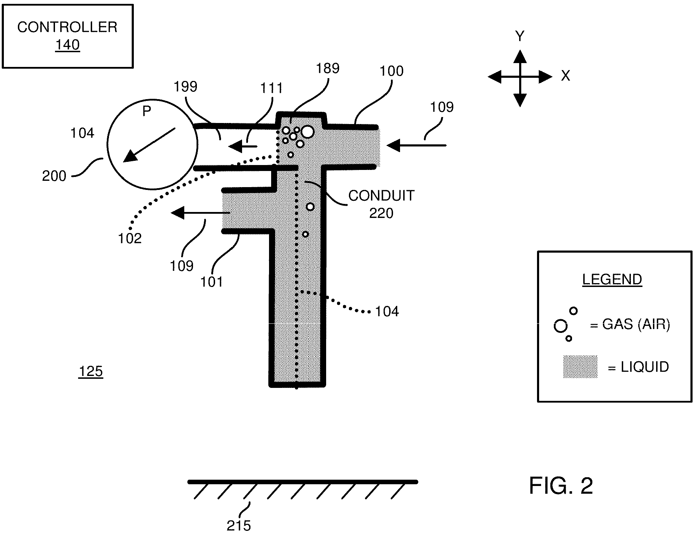

FIG. 2 is an example diagram illustrating implementation of an air elimination assembly as discussed herein.

As shown in FIG. 2, an AEA (i.e., air elimination assembly or air elimination filter) design is enhanced to include a membrane such as a hydrophilic membrane. For example, in one configuration, the input port 100 (such as liquid inlet, liquid input port, channel, conduit, etc.) and output port 101 (such as liquid outlet, liquid output port, channel, conduit, etc.) are separated by a filter membrane 104 (such as a hydrophilic filter membrane or other suitable entity). Once wetted by the infusion liquid (a.k.a., liquid 109), the hydrophilic filter membrane 104 uses surface tension to prevent air in the liquid path from passing through to the outlet 101. The filter membrane 104 also has a given pore size that will filter out any particles that are in the liquid 109 (i.e., liquid). Pore sizes of example hydrophilic filters for use herein (membrane 104) typically range from 0.2 to 5 um in diameter, although any pore size can be used. Presence of the second membrane 104 in the conduit 220 (a.k.a., chamber, pathway, etc.) also prevents passage of the gas 189 from the liquid 109 to the output port 101.

In some cases, the operation of filtering particles from the liquid (such as liquid 109) is desirable, such as during a condition in which the particles are present in the liquid 109 based on a contaminant from a respective manufacturing process that produces the liquid 109. In other cases, particles in the liquid 109 might be necessary, such as red blood cells or therapeutic pharmaceutical particles, which cannot be removed from the liquid.

As further shown, the air elimination assembly 125 also includes a connection to an external pressure control source 200 (such as a pressure controller). The pressure control resource 200 can be configured to regulate or maintain pressure on the gas output port 199 such that the air side (gas output port 199) of the vent membrane 102 is lower than the pressure of the liquid 109 in the liquid path (conduit 220 between the input port 100 and the output port 101). This ensures that there is a pressure differential across the vent membrane 102 (such as between the input port 100 and the gas output port 199) that will cause bubbles (such as gas 189) to be vented out of the liquid path of liquid 109 passing from the input port 100 to the output port 101 of the air elimination assembly 125.

Note further that the air elimination assembly 125 further prevents air from passing back from gas output port 199 through the vent membrane 102 and into the liquid path (conduit 220) of passing liquid 109. The reduced pressure at the gas output port 199 as controlled by pressure control source 200 can be implemented via any of one or more technologies such as a vacuum pump, a centralized hospital vacuum system, a depressurized air tank, etc.

The air elimination assembly 125 as discussed herein therefore includes membrane 104 through which the liquid 109 passes from the input port 100 and conduit 220 and membrane 104 to the output port 101. As previously discussed, in further examples, the air elimination assembly 125 includes a membrane 102 through which the gas 111 (such as from bubbles 189) in the liquid 109 passes to the gas output port 199. In this example, the air elimination assembly 125 is parallel to the y-axis (up-down direction). In such an instance, via buoyancy, the bubbles such as gas 189 flow to the top of the conduit 220 of the air elimination assembly 125.

Thus, in accordance with one configuration, the membrane 102 is disposed at a higher position above a ground level 215 in the air elimination assembly 125 than the membrane 104; a buoyancy of the gas 189 causes at least a portion or all of the gas 189 to flow from a level of the second membrane 104 to a level of the first membrane 102; the gas 189 passes through the membrane 102 as gas 111 outputted from the gas output port 199.

Thus, as discussed herein, the input port 100 of the air elimination assembly 125 receives liquid 109. A controller 140 (or other suitable entity) controls a magnitude of pressure at the gas output port 199 of the air elimination assembly in order to expel gas 111 (such as air removed) from the liquid 109 passing from the input port 100 to the output port 101. Controlling the magnitude of pressure at the gas output port 199 with respect to the pressure at the input port 100 causes the gas 189 in the liquid 109 to pass through the membrane 102 of the air elimination assembly 125 to the gas output port 199 as gas 111. As previously discussed, the gas output port 199 outputs the gas 111 passing through the membrane 102. The liquid output port 101 of the air elimination assembly 125 outputs the liquid 109 (now void of gas 189) along a respective liquid pathway in a direction toward the recipient 108.

The controller 140 can be configured to set the pressure at the gas output port 199 to be lower than a magnitude of pressure of the liquid 109 inputted to the input port 100 and pressure of the liquid 109 in the conduit 220. The difference between the magnitude of pressure at the gas output port 199 and pressure of the liquid 109 at the input port 100 or in conduit 220 can be any suitable value. In one example, the difference between the magnitude of pressure at the gas output port 199 and the magnitude of pressure of the liquid 109 inputted to the input port 100 or pressure of the liquid 109 in the air elimination assembly 125 is greater than 1 PSI (Pound per Square Inch).

In further examples, the controller 140 controls the magnitude of pressure at the gas output port 199 based on one or more parameters or attributes such as attributes of the membrane 102, attributes of the liquid 109, etc.

In one example, the attributes of the liquid 109 itself can be used by an operator of the controller 140 as a basis to control a magnitude of pressure at the gas output port 199. Such parameters may include one or more of: i) a viscosity of the liquid 109, and ii) a surface tension of the liquid 109, etc.

Additional attributes that can be used by the operator of the controller 140 as a basis to control a magnitude of pressure at the gas output port 199 include one or more of: a type of material used to fabricate the membrane 102, a pore size of the membrane 102, water-entry pressure associated with the membrane 102, etc.

Note that the controller 140 can be further configured to, if desired, vary a magnitude of the negative pressure applied at the gas output port 199 of the air elimination assembly 125 during conveyance of the liquid 109 through the air elimination assembly 125 from the liquid input port 100 to the liquid output port 101.

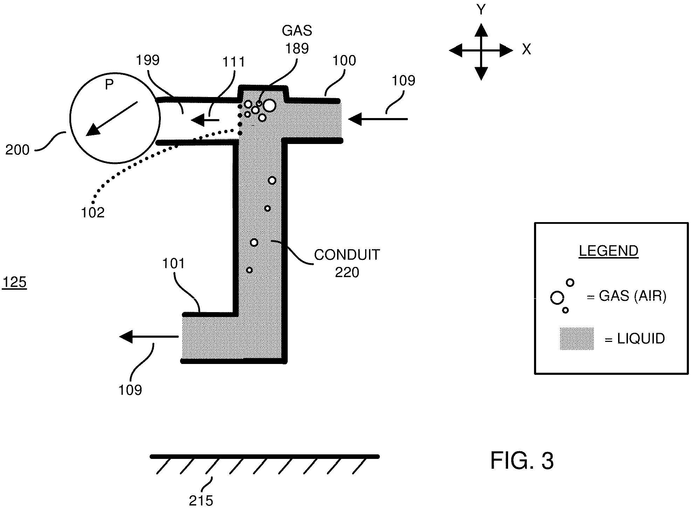

FIG. 3 is an example diagram illustrating implementation of an air elimination assembly as discussed herein.

In this example the negative pressure control source 200 is included on the air side (gas output port 111) of the vent membrane 102 but the hydrophilic filter membrane 104 from FIG. 2 has been removed from conduit 220.

Removal of the hydrophilic filter membrane 104 enables the air elimination assembly 125 to be used with liquids that are incompatible with filtration (such as blood products), while still allowing air (gas 189) in the liquid 109 to be vented from the liquid path and liquid 109 through the vent membrane 102 to the gas output port 199.

In this example, the liquid output port 101 (a.k.a., outlet) is disposed lower such that the output port 101 is disposed substantially at the bottom of the air elimination assembly 125 near the ground reference 215. Disposing the liquid output port 101 at or near the bottom of the AEA 125 and the gas output port 199 and the input port 100 further away from the ground reference 215 allows a buoyancy effect to drive any gas 189 such as air bubbles in the liquid path of liquid 109 up toward the vent (membrane 102). This prevents the gas 189 from flowing to and out of the output port 101 of the air elimination assembly 125.

Thus, in one example, the air elimination assembly 125 includes a membrane 102 through which the gas 189 in the liquid 109 passes (as gas 111) to the gas output port 199. As further shown in this example, the gas output port 199 is disposed at a higher position above a ground level 215 in the air elimination assembly 125 than the liquid output port 101. As previously discussed, a buoyancy of the gas 189 causes at least a portion or all of the gas 189 to a level of the gas output port 199. The negative pressure applied to the gas output port 111 by the pressure control source 200 (where the pressure of the gas output port 111 is less than or is negative with respect to the pressure of the liquid 109 in the conduit 220 or at the input port) causes the gas 189 to pass through the membrane 102.

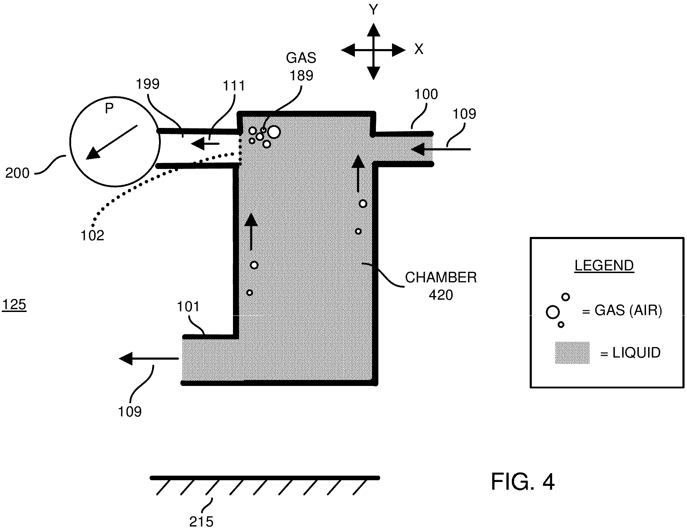

FIG. 4 is an example diagram illustrating implementation of an air elimination assembly as discussed herein.

The air elimination assembly 125 in FIG. 4 is an extension of the air elimination assembly 125 shown in FIG. 3, with the addition of a so-called expansion chamber 420 in between the input port 100 and the output port 101. The expansion chamber 420 can be configured such that the cross-sectional area of the liquid flow path (such as orthogonal to the flow of liquid 109) is substantially larger in the expansion chamber 420 than it is in the input port 100. Assume that axis Z is orthogonal out of the drawing and with respect to axis X and axis Y. The liquid input port 100 has a smaller cross-sectional area in the Y-Z plane than the cross-sectional area (X-Z plane) of the liquid flow path in the chamber 420. The liquid input port 100 may also have a smaller cross-sectional area in the Y-Z plane than the cross-sectional area (Y-Z plane) of the liquid flow path in the output port 101.

In a typical case, the area (in X-Z plane) of the flow path in the expansion chamber 420 (a.k.a., conduit) would be greater in cross-section area than that of the input port 100 flow path. Note further that the increase in cross-sectional flow path area in the chamber 420 (with respect to the input port 100 or output port 101) results in a corresponding decrease in liquid 109 velocity through the expansion chamber 420 from the input port 100 to the output port 101. In other words, differences in cross sectional areas, the flow velocity of liquid 109 through the chamber 420 is substantially less than a flow velocity of the liquid 109 through the input port 100 or a flow velocity of the liquid 109 through the output port 101.

Note that the high velocity liquid flow can entrain small air bubbles and the bubbles can be swept along with the liquid flow, despite buoyant forces trying to push them upwards towards the gas output port 199. Via the large cross-section in the X-Z plane as provided by the chamber 420, the velocity of liquid 109 is suitably decreased along the Y-axis, resulting in the buoyant forces on the gas 189 in the liquid 109 overcoming the drag force of the liquid flow of liquid 109 passing downward through the chamber 420 from the input port 100 to the output port 101. Thus, bubbles (gas 189) float (move) to the top of the air elimination assembly 125 and are vented out through the vent membrane 102 (a.k.a., filter) to the gas output port 199, where the gas 111 (from gas 189) is expelled from the air elimination assembly 125. Thus, the expansion chamber 420 as discussed herein provides additional assurance that all bubbles in liquid 109 will be removed from the liquid path even without the use of a hydrophilic filter membrane 104 (FIG. 2).

Accordingly, examples herein include a chamber 420 disposed in the air elimination assembly 125 between the input port 100 and the output port 101. The chamber 420 has a cross-section larger than a cross section of the input port 100 such that the velocity of the liquid 109 passing from the input port 100 to the output port 101 is substantially less (such as 2 times less, 5 times less, 10 times less, etc.) than the velocity of the liquid 109 received through the input port 100.

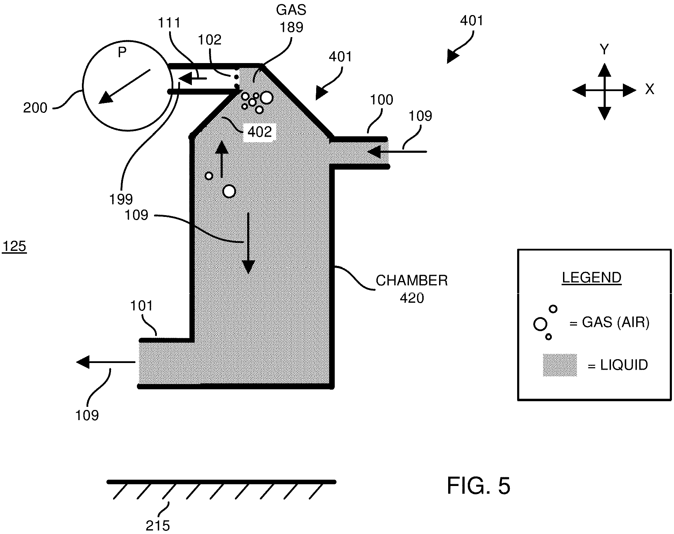

FIG. 5 is an example diagram illustrating implementation of an air elimination assembly as discussed herein.

As shown, FIG. 5 further illustrates how the expansion chamber 420 can be configured to include any number of tapered walls, such as tapered wall 401, tapered wall 402, etc. The tapered walls 401 and 402 (such as a cone, pyramid, or any other shape) help guide gas 189 such as air bubbles toward the vent membrane 102 as they float (such as caused by buoyancy) to the top (such as away from ground 215 of the expansion chamber 420 as shown.

Accordingly, the air elimination assembly 125 as discussed herein can be configured to include a chamber 520 disposed between the input port 100 and the output port 101. The chamber 520 includes a respective tapered pathway extending from the chamber 520 to the gas output port 199. As previously discussed, the gas output port 111 can be disposed at a higher level than the input port 100 and/or the output port 101 with respect to ground level 215.

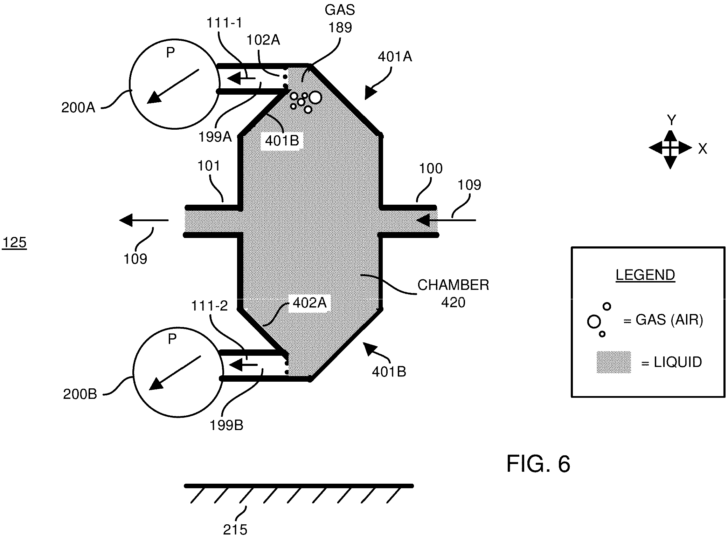

FIG. 6 is an example diagram illustrating implementation of an air elimination assembly as discussed herein.

As shown in FIG. 6, note that the expansion chamber 420 and corresponding air elimination assembly can be modified in a number of ways to provide operation (gas removal) in any orientation. One such method is to move input port 100 and output port 101 to a substantially central location within the expansion chamber 420 and include two or more vents 102 (gas output ports) at outer extremities of the chamber 400 as shown in FIG. 6 such that at least one vent (such as vent membrane 102A or vent membrane 102B) is always above the input port 100 and/or output port 101 in any orientation of rotation of the air elimination assembly 125 about axis X.

Accordingly, in one example, the air elimination assembly 125 includes a first gas output port 199A and a second gas output port 199B. As previously discussed, the chamber 400 is disposed between the input port 100 and the output port 101. The air elimination assembly 125 and corresponding chamber 420 include a first tapered pathway (such as via chamber walls 401A and 401B) extending all or a portion of the way from the input port 100 of chamber 420 to the first gas output port 199A through which gas 111-1 is expelled. The air elimination assembly 125 and corresponding chamber 400 includes a second tapered pathway (such as via chamber walls 402A and 402B) extending from the input port 100 of chamber 420 to the second gas output port 199B through which gas 111-2 is expelled.

The input port 100 and/or the output port 101 are disposed between the first tapered pathway and the second tapered pathway.

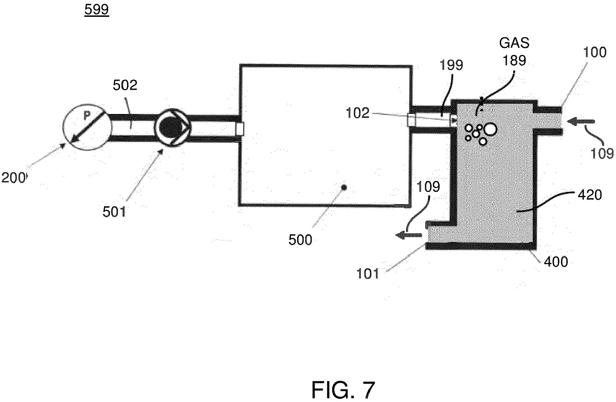

FIG. 7 is an example diagram illustrating implementation of an air elimination assembly including a valve as discussed herein.

In certain situations, a negative pressure source might not be perpetually available to apply a negative pressure to gas output port 199. In these cases, it may be advantageous for the air elimination assembly 125 to include a pressure storage reservoir (such as chamber 500) as part of the air elimination assembly 125.

As shown in FIG. 7, the air elimination assembly 125 can be configured such that the air side of vent membrane 102 (i.e., of gas output port 199) is connected to an air chamber 500 (a.k.a., reservoir) made up of a portion of the air elimination assembly housing. The chamber 500 can be configured to connect to the negative pressure control source 200 with a valve 501 positioned between the reservoir 500 and the pressure control source 200. Valve 501 can be a check valve, electronically controlled valve, manually activated valve, etc.

In one example, the valve 501 is a check valve allowing all or a portion of the gas in the air chamber 500 to be exhausted from output port 502 to the (negative) pressure control source 200. The (check) valve 501 prevents air (gas) in output port 502 from passing through the output port 502 to the air chamber 500, maintaining a negative pressure in the air chamber 500 and gas output port 199.

Thus, the pressure control source 200 is attached to the output port 502 of the valve 501. In general, the pressure control source 200 applies a negative pressure to the output port 502. In other words, the pressure control source 200 applies a magnitude of pressure to the outlet port 502 that is lower than the pressure in the air chamber 500 and gas output port 199. In such an instance, opening of the valve 501 causes all or a portion of gas 189 in the chamber 500 to be expelled. Closing of the valve 501 again causes chamber 500 and gas output port 199 to be held at a magnitude of pressure that is below the pressure of liquid 109 in chamber 420 or input port 100. Thus, a closed position of the valve 501 (or directionality of the check valve 501) prevents gas being supplied by output port 502 from flowing into the chamber 500 again. The air chamber 500 then provides a negative pressure to the gas output port 199 to provide gas reduction in the chamber 420 in a manner as previously discussed. In such an instance, a magnitude of pressure at gas output port 199 is suitably lower than the pressure of liquid 109 in the chamber 420. If desired, the pressure control source 200 can be configured to change the negative pressure in chamber and gas output port 199 to any suitable value.

In accordance with further examples, the pressure control source 200 can be configured to attach and detach from the valve 501 during use of the air elimination assembly 125. In this fashion, in a manner as previously discussed, the pressure control source 200 can be used to charge the air chamber 500 (a.k.a., pressure reservoir) to a negative pressure with respect to the ambient pressure and/or pressure at the input port 100. The negative pressure is then retained in the air chamber 500 by the valve 501 (such as closed) even if the pressure control source 200 is disconnected from the gas output port 199, or if the pressure source of gas is adjusted to a higher pressure level. In one example, the negative pressure in the air chamber 500 draws gas 189 such as bubbles out of the vent membrane 102 (vent) even when pressure control source 200 is not present.

In further examples, the air chamber 500 is sized such that the air volume of the reservoir is substantially larger than the volume of air expected to be vented from the liquid path before the air chamber 500 is recharged by pressure control source 200. Having the reservoir 500 substantially larger than a total volume of the air (gas 189) to be vented ensures that the pressure in the air chamber 500 will remain suitably low (e.g. far enough below the liquid pressure of liquid 109 in the chamber 400) as gas 189 passes through the membrane 102 and gas output port 199 to the reservoir 500.

As stated previously, the pressure control source 200 can be implemented via any number of one or more technologies. With the inclusion of the air chamber 500 and the valve 501, additional, intermittent pressure sources can be utilized. For example, a clinician operating the liquid pump as discussed herein may attach a syringe to the output port 502 of the valve 501, draw back the plunger of the syringe to charge the air chamber 500 to a negative pressure, disconnect the syringe, and then use the air elimination assembly 125 to vent air gas 189 from the liquid 109 of an infusion therapy being delivered to recipient 108 such as a patient.

The pressure control source for the air elimination assembly may be a pneumatically driven IV (Intravenous) infusion pump. Infusion pumps that utilize pneumatic pressure to drive liquid to a patient are known in the art and provide many advantages over typical mechanically driven infusion pumps. Note that examples herein include a pneumatically driven diaphragm pump where the durable equipment portion of the system (the "pump") provides pneumatic pressure and mechanical actuators that interface with a disposable cassette that forms a portion of the liquid path connecting to a liquid source and delivering liquid to a recipient. The cassette 104 can be configured to include a pneumatically-driven intermediate pumping chamber (IPC) that moves liquid from the input port to the output port. The pump further can be configured to supply negative air pressure to the IPC to draw liquid in from the input port 100, and then the pump supplies positive air pressure to the IPC to push liquid to the output port 101.

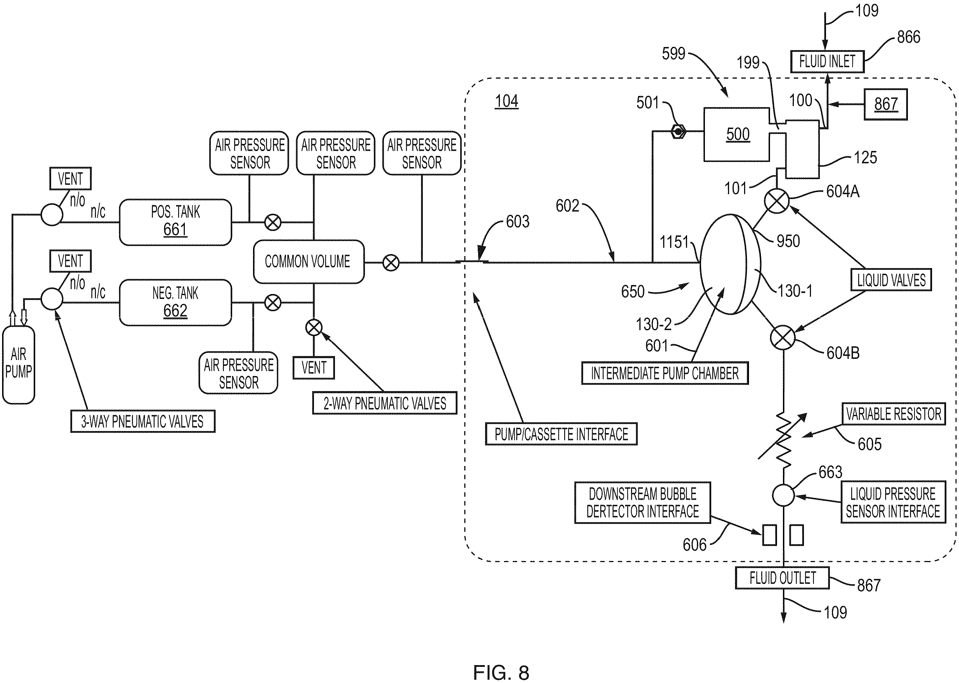

FIG. 8 is an example diagram illustrating a liquid delivery system as discussed herein.

Via system 599 (such as implementation of FIG. 7 as previously discussed), examples herein include a method of controlling the magnitude of pressure at the gas output port 199 including: via pressure monitor 867, monitoring a magnitude of the pressure of the liquid 109 inputted to the input port 100; deriving or determining a pressure value from the magnitude of the pressure of the liquid 109 inputted to the input port 100, the derived pressure value lower than a pressure of the liquid at the input port 100; and setting the pressure at the gas output port 199 to be the derived (appropriate or desirable) pressure value via negative pressure provided to the gas output port 199 from the chamber 500.

As shown, FIG. 8 includes an air elimination assembly 125 disposed in a cassette 104 of a pneumatically driven liquid pump to provide automatic, pressure-assisted removal of air from the liquid path.

More specifically, in this example, the AEA assembly 600 is integrated into the disposable cassette 104 of the liquid delivery system. The cassette 104 includes air elimination assembly 125, diaphragm pump 650, variable liquid flow resistor 605, liquid pressure sensor 663, and downstream bubble detector interface 606. Note that the AEA 600 can be directly integrated as appropriately configured features of the same components that make up the rest of the pumping cassette or could be a separate assembly of components that is attached to the cassette during the manufacturing process.

In one example, there is a pneumatic coupling 603 between the pump portion (left portion of FIG. 8 with respect to cassette 104) and the cassette 104 to provide positive and negative pressurized air to the chamber 130-2 of diaphragm pump 650 as well as chamber 500 of the cassette 104. The chamber 500 includes a valve 501 allowing air to be expelled from chamber 500 into the tube 602. The tube 602 such as a pneumatic connection connects the gas pressure from the coupling 603 to both the intermediate pump chamber (IPC) 601 and the output port of the valve 501 associated with the AEA 125. When the controller applies negative pressure to the coupling 603, the negative pressure draws liquid into the IPC 601 and also charges (evacuates gas from) the pressure reservoir (chamber 500) associated with the air elimination assembly 125 to a negative pressure so that air reaching the vent (gas output port 199) will be removed from the liquid path in the air elimination assembly 125 between the input port 100 and output port 101 of the air elimination assembly 125. When the controller applies positive pressure to the coupling 603, that gas pressure is communicated to the IPC and can be used to push liquid out of the IPC 601 to the liquid output port downstream to and through valve 604B. At the same time, the valve 501 within the AEA 600 blocks the positive pressure from reaching the AEA pressure reservoir (chamber 500), so that reservoir (chamber 500) stays charged at the negative pressure and continues venting air bubbles from the liquid path associated with air elimination assembly 125.

Note that the sequence of pressure application can occur in any number of ways, and the described sequence is only one example. One other example is that the pump can be configured to close the liquid valves 604A and 604B to prevent liquid flow into or out of the IPC of diaphragm pump (isolating the IPC of diaphragm pump 650) before negative pressure is applied to the pneumatic coupling 603. In such a configuration, a more negative pressure can be used to charge the AEA 600 reservoir. Then the pump can be configured to apply a less negative pressure to the pneumatic coupling 603 prior to opening the inlet liquid valve 604A to allow liquid (liquid 109) to flow into the IPC 601 (chamber 130-2). This sequence ensures that the air elimination assembly 600 would be at a lower pressure than the liquid (liquid 109) being drawn into the IPC 601 so that bubbles moving past the vent (gas output port 199) as the IPC fills will be drawn out of the liquid path.

FIG. 8 further shows an air elimination assembly 600 incorporated into the liquid path upstream of the IPC 601 (130-2) between the liquid input port and the inlet valve 604A. However, one or more instances of an air elimination assembly could be incorporated at any number of locations in the liquid path of the cassette from the liquid inlet 866 to the liquid outlet 867 of the cassette 104, depending on where the need to vent bubbles is most prevalent in a given liquid delivery system. Further, note that the cassette 104 can be configured to include multiple air elimination assemblies to vent bubbles at multiple locations in the liquid pathway between the inlet 866 and the outlet 867.

Accordingly, examples herein include controlling operation of a diaphragm pump 650 to deliver the liquid 109 through the air elimination assembly 125 to a recipient 108. The operation of the diaphragm pump 650 may include application of positive pressure and negative pressure to a chamber 130-2 of the diaphragm pump 650; and utilizing the negative pressure (from negative tank 662 or chamber 600 such as negative pressure gas) associated with negative tank 662 to set the magnitude of pressure at the gas output port 199 of the air elimination assembly 125. As previously discussed, if desired, the controller 140 can be configured to vary a magnitude of the pressure applied at the gas output port 199 of the air elimination assembly 125 during conveyance of the liquid 109 through the air elimination assembly 125 from the input port 100 to the output port 101.

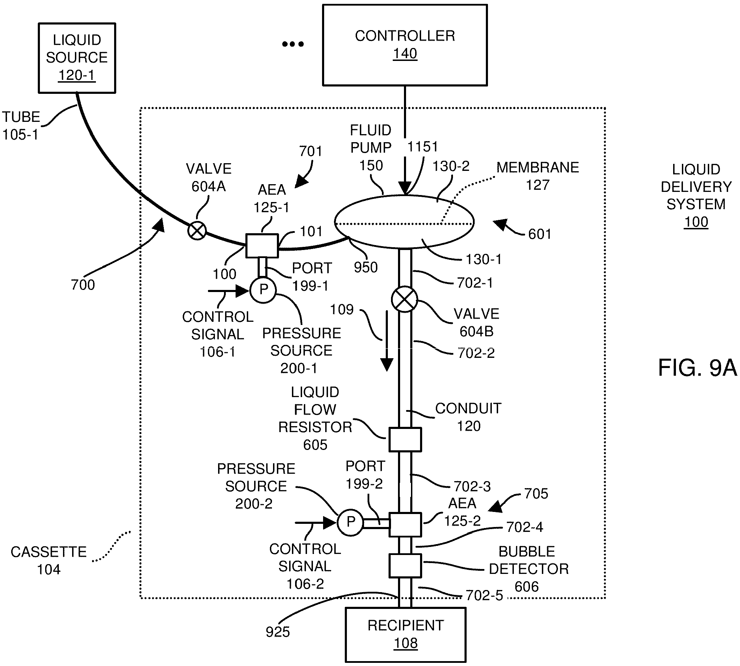

FIG. 9A is an example diagram illustrating implementation of an air elimination assembly in a liquid pathway as discussed herein.

The liquid delivery system 100 in FIG. 9A shows multiple locations where it is useful to include one or more air elimination assemblies 125 to a pneumatically driven pump system.

One embodiment for integration of air elimination assemblies into a pneumatically driven pump cassette, there are two air elimination assemblies included in the disposable cassette 104.

In this example the air elimination assembly 125-1 is located at position 701 between valve 604A and chamber 130-1 .

Further in this example, the second air elimination assembly 125-2 is located at position 705, immediately upstream of the bubble detector 606 located at or near the output port 925 of the cassette, and downstream of the liquid flow resistor 605 (such as a controlled variable flow resistor controlling a flow of the liquid).

In most liquid pumps, the operation of moving liquid 109 through the system can cause additional air bubbles to precipitate out of the solution. This can be due to warming of the liquid (by the pump, or just from ambient temperature), mechanical agitation of the liquid by the pump, or by pressure drops across restrictions in the liquid path

Positioning of the second air elimination assembly (at location 705) as close to the output port 925 such as near bubble detector 606 of the cassette 104 as possible, but upstream of the bubble detector 606, and downstream of the liquid flow resistor 605 allows for venting of the most amount of air (gas) that may have come out of solution (liquid 109) during pumping, while still maintaining the backup protection of the bubble detector 606 which is configured to sound an alarm if any gas is detected by the bubble detector 606.

Thus, as shown in FIG. 9A, liquid delivery system 100 includes liquid pump 150 (such as a diaphragm pump), cassette 104, controller 140, and liquid source 120-1.

Liquid source 120-1 is connected to liquid pump 150 via the tube 105-1 (conduit, liquid pathway, inlet, etc.).

Note that liquid pump 150 liquidcan be any suitable type of device capable of pumping liquid through conduit 120 to the recipient 108 (such as a diaphragm pump, peristaltic liquid pump, piston pump, etc.).

As previously discussed, the liquid pump 150 can be configured as a diaphragm pump including flexible membrane 127 (such as made form elastically deformable material. layer of rubber, etc.). Flexible membrane 127 divides the liquid pump 150 into chamber 130-1 and chamber 130-2. The controller 140 can be configured to repeatedly monitor a pressure of chamber 130-2 to determine an amount of liquid in the chamber 130-1. The controller 140 uses this information to control a flow rate of liquid inputted into conduit 120.

In addition to controlling gas pressure applied to chamber 130-2 to control a flow of liquid 109 inputted into/through conduit 702 (such as portions including conduit 702-1, conduit 702-2, conduit 702-3, conduit 702-4, and conduit 702-5), the controller 140 controls valve 604A and valve 604B at appropriate times to draw respective liquid into the chamber 130-1 and then expel the liquid 109 in the chamber 130-1 into and through the conduit 702 downstream to the recipient 108.

To draw liquid 109 from liquid source 120-1 into the chamber 130-1, while valve 604B is controlled by controller 140 to be closed and valve 604A is open, the controller 140 applies a negative pressure to the port 1151 of the chamber 130-2 to draw liquid 109 from source 120-1 into the chamber 130-1. As previously discussed, the air elimination filter 125-1 removes any gasses in the liquid 109 passing through it via gas output port 199-1.

Subsequent to filling the chamber 130-1, while the controller 140 controls valve 604A to a respective closed state and controls the valve 604B to an open state, the controller 140 applies a positive gas pressure to chamber 130-2, causing the liquid 109 in chamber 130-1 to flow downstream into and though conduit 702-1.

Via control of valves 604A and 604B, and gas pressure in chamber 130-2, the controller 140 precisely controls delivery of the primary liquid 109 from liquid source 120-1 at the desired rate into conduit 702-1 as well as further downstream. The controller 140 repeats this control cycle (drawing liquid 109 into chamber 130-1 and then expelling it) to deliver a desired amount of liquid 109 from liquid source 120-1 into the conduit 702.

In this example, the cassette 104 includes air elimination assembly 125-1 and air elimination assembly 125-2. The air elimination assembly 125-1 removes any gas 189 from the liquid 109 passing from source 120-1 through a combination of tubing 105-1, air elimination assembly 125-1, and valve 604A to the chamber 130-1.

The conduit 702 further includes air elimination assembly 125-2. The diaphragm pump 131 expels liquid 109 from the chamber 130-2 through a pathway including conduit 702-1, valve 640B, conduit 702-2, liquid flow resistor 605 (controlled by controller 140), conduit 702-3, air elimination filter 125-2, bubble detector 606, and conduit 702-5 to the recipient 108.

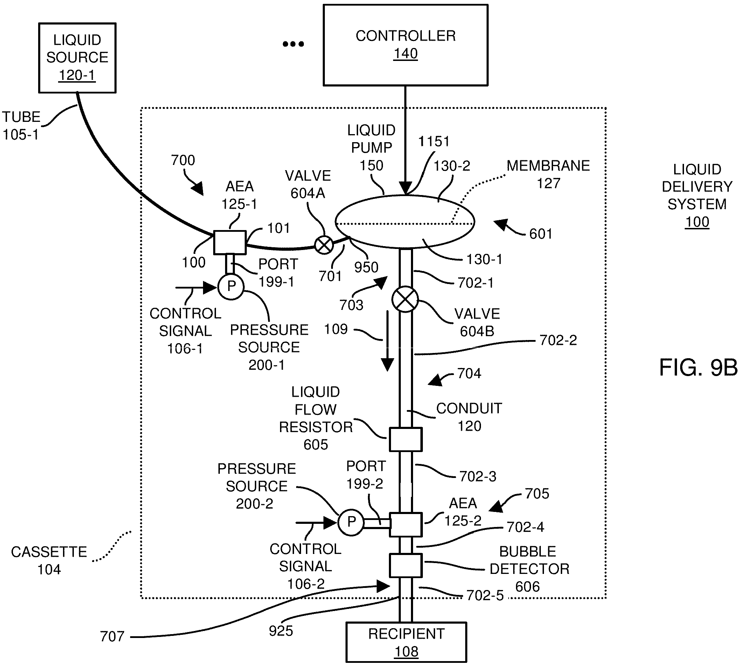

FIG. 9B is an example diagram illustrating implementation of an air elimination assembly in a liquid pathway as discussed herein.

In this example, the first air elimination assembly 125-1 is located at position 700, upstream of the input port valve 604A. In other words, the air elimination assembly 125-1 is disposed between the liquid source 120-1 and the valve 604A. Implementation at this location 700 allows bubbles in the inlet tube 105-1 to be vented out of the liquid path while the inlet valve 604A is closed and the IPC 601 is pumping liquid 109 in chamber 130-1 of the liquid pump 150 (diaphragm pump) to the output port 925 to recipient 108.

Note further that a respective instance of the air elimination assembly 125 can be located at any one or more location in the cassette 104 such as at location 700 between the liquid source 120-1 and the valve 604A, at location 701 between the valve 604A and liquid pump 150, at location 703 between the liquid pump 150 and valve 604B, at location 704 between the valve 604B and flow resistor 705, at location 705 between the flow resistor 705 and the bubble detector 606, at location 707 between the bubble detector 606 and the output port 925, and so on.

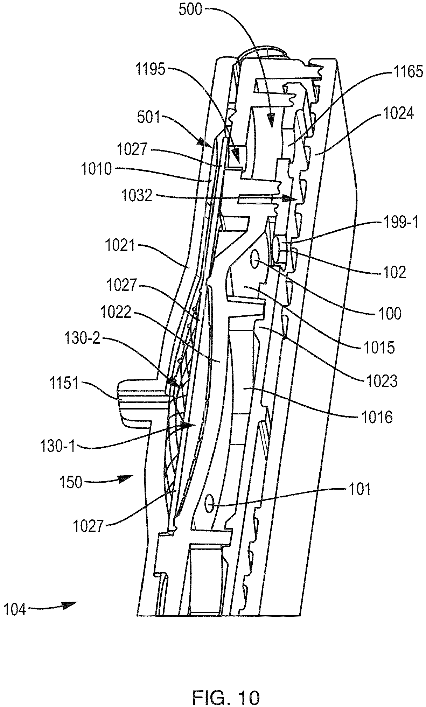

FIG. 10 is an example diagram illustrating a cutaway view of a cassette assembly including elements as discussed herein.

In this example, the cassette 104 includes multiple layers of structures/material and components to provide chambers, ports, pathways, etc., supporting functions as previously discussed. The air elimination assembly 125-1 is integrated in the cassette 104, which includes an integrated diaphragm pump to pump the liquid 109.

For example, a portion of the structure 1021 and structure 1022 creates a liquid pump 150 (such as a diaphragm pump) including chamber 130-1 and chamber 130-2. The liquid pump 150 includes a diaphragm membrane 127 separating the chamber 130-1 and the chamber 130-2. As previously discussed, the controller 140 controls application of negative pressure and positive pressure at different times to the port 1151 results in drawing liquid 109 into the chamber 130-1 and expelling the liquid 109 in the chamber 130-1 downstream to a respective conduit for delivery to a recipient 108.

As previously discussed, the air elimination assembly 125-1 can be configured to include a respective chamber 500 to store a respective negative pressure applied to gas output port 199-1. In this example, the chamber 500 is fabricated via walls of the structure 1022 and structure 1023.

In this example, the diaphragm membrane 127 extends between the structure 1021 and the structure 1022 to port 1195 such that the interface between the diaphragm membrane 127 and an edge of the port 1195 produces a respective valve 501. As previously discussed, when the controller 140 applies a negative pressure to port 1151, the negative pressure in chamber 130-2 extends along the open passageway 1010 (such as airspace between structure 1021 and the membrane 127) to valve 501 at the port 1195 (such as a volcano shaped tip of port 1195 in contact with the diaphragm membrane 127). When negative pressure is applied, the diaphragm membrane 127 flaps open to exhaust gas from chamber 500, producing a negative pressure in the chamber 500. The negative pressure in chamber 500 passes through port 1165 and passageway 1032 between the structure 1023 and structure 1024 to the gas output port 199-1 and corresponding filter 102.

The liquid 109 passes from input port 100 through chamber 1015 and chamber 1016 (formed via structure 1022 and structure 1023) to the output port 101. As previously discussed, the gas output port 199-1 can be configured to include a filter 102 allowing gas to pass from liquid 109 in the chamber 1009 to pass to the gas output port 199-1. The liquid delivery system can be configured to implement vertical orientation of the cassette in the liquid pump 157 such that any bubbles precipitated from the liquid 109 passing through the chamber 1015 and chamber 1016 (from input port 100 to output port 101) move in a direction from the output port 101 to the gas output port 199-1 where the corresponding gas 189 in liquid 109 is expelled through the filter 102 and the gas output for 199-1 into the pathway 1032. Accordingly, any gas 189 in liquid 109 passing through chamber 1015 and chamber 1016 can be expelled out of the gas output port 199-1 prior to entering the chamber 130-2.

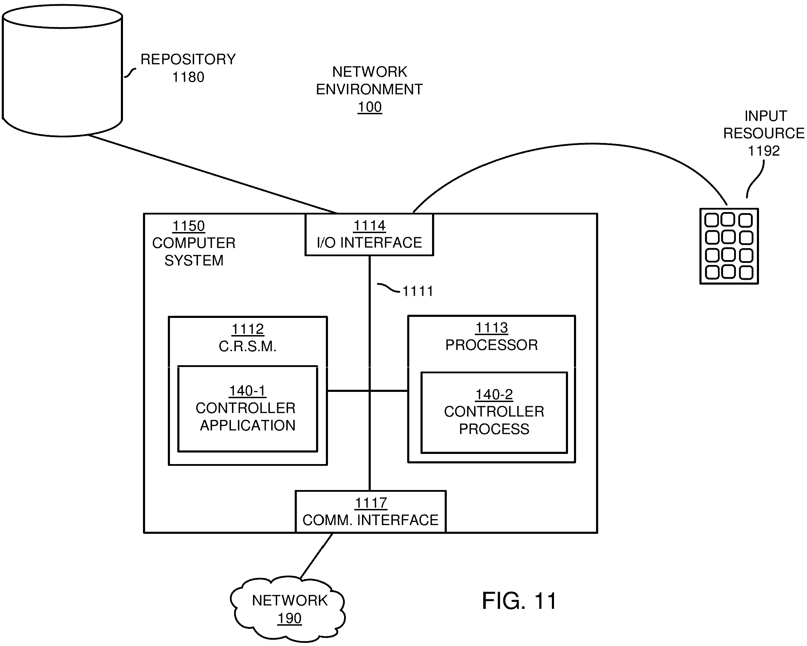

FIG. 11 is an example block diagram of a computer device for implementing any of the operations as discussed herein as discussed herein.

In one example, liquid management system includes one or more computer systems similar to computer system 1150 to execute management application/process associated with the controller 140.

As shown, computer system 1150 of the present example includes an interconnect 1111, a processor 1113 (such as one or more processor devices, computer processor hardware, etc.), computer readable storage medium 1112 (such as hardware storage to store data), I/O interface 1114, and communications interface 1117.

Interconnect 1111 provides connectivity amongst processor 1113, computer readable storage media 1112, I/O interface 1114, and communication interface 1117.

I/O interface 1114 provides connectivity to a repository 1180 and, if present, other devices such as a playback device, display screen, input resource 1192, a computer mouse, etc.

Computer readable storage medium 1112 (such as a non-transitory hardware medium) can be any hardware storage resource or device such as memory, optical storage, hard drive, rotating disk, etc. In one example, the computer readable storage medium 1112 stores instructions executed by processor 1113.

Communications interface 1117 enables the computer system 1150 and processor 1113 to communicate over a resource such as network 190 to retrieve information from remote sources and communicate with other computers. I/O interface 1114 enables processor 1113 to retrieve stored information from repository 1180.

As shown, computer readable storage media 1112 is encoded with controller application 140-1 (e.g., software, firmware, etc.) executed by processor 1113. Controller application 140-1 can be configured to include instructions to implement any of the operations as discussed herein.

During operation of one example, processor 1113 (e.g., computer processor hardware) accesses computer readable storage media 1112 via the use of interconnect 1111 in order to launch, run, execute, interpret or otherwise perform the instructions in controller application 140-1 stored on computer readable storage medium 1112.

Execution of the controller application 140-1 produces processing functionality such as controller process 140-2 in processor 1113. In other words, the controller process 140-2 associated with processor 1113 represents one or more aspects of executing controller application 140-1 within or upon the processor 1113 in the computer system 1150.

Those skilled in the art will understand that the computer system 1150 can include other processes and/or software and hardware components, such as an operating system that controls allocation and use of hardware resources to execute management application 140-1.

In accordance with different examples, note that computer system may be any of various types of devices, including, but not limited to, a wireless access point, a mobile computer, a personal computer system, a wireless device, base station, phone device, desktop computer, laptop, notebook, netbook computer, mainframe computer system, handheld computer, workstation, network computer, application server, storage device, a consumer electronics device such as a camera, camcorder, set top box, mobile device, video game console, handheld video game device, a peripheral device such as a switch, modem, router, or in general any type of computing or electronic device. In one nonlimiting example, the computer system 1150 resides in liquid delivery system 100. However, note that computer system 1150 may reside at any location or can be included in any suitable one or more resources in network environment to implement functionality as discussed herein.

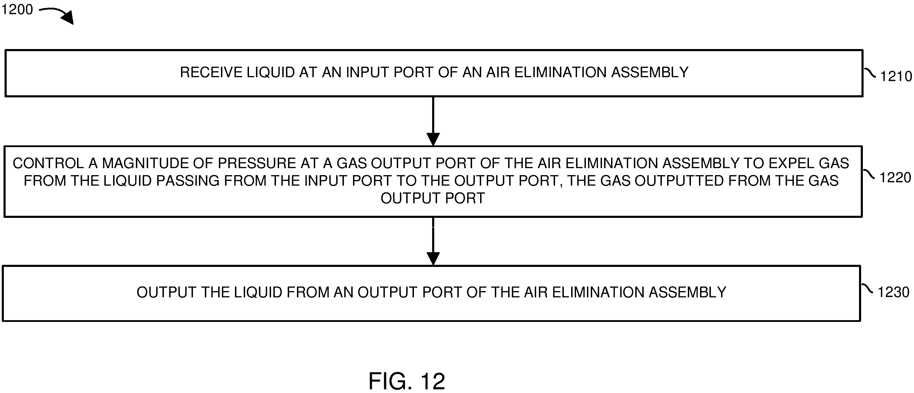

Functionality supported by the different resources will now be discussed via flowcharts in FIG. 12. Note that the steps in the flowcharts below can be executed in any suitable order.

FIG. 12 is a flowchart 1200 illustrating an example method as discussed herein. Note that there will be some overlap with respect to concepts as discussed above.

In processing operation 1210, the air elimination assembly 125 receives liquid 109 at an input port 100.

In processing operation 1220, the controller 140 controls a magnitude of pressure at a gas output port 199 of the air elimination assembly 125 to expel gas 189 from the liquid 109 passing from the liquid input port 100 to the liquid output port 101. The gas is outputted from the gas output port 199 of the air elimination assembly 125.

In processing operation 1230, the air elimination assembly 125 outputs the liquid 109 from the liquid output port 101.

Note again that techniques herein are well suited for use in liquid delivery systems. However, it should be noted that examples herein are not limited to use in such applications and that the techniques discussed herein are well suited for other applications as well.

Based on the description set forth herein, numerous specific details have been set forth to provide a thorough understanding of claimed subject matter. However, it will be understood by those skilled in the art that claimed subject matter may be practiced without these specific details. In other instances, methods, apparatuses, systems, etc., that would be known by one of ordinary skill have not been described in detail so as not to obscure claimed subject matter. Some portions of the detailed description have been presented in terms of algorithms or symbolic representations of operations on data bits or binary digital signals stored within a computing system memory, such as a computer memory. These algorithmic descriptions or representations are examples of techniques used by those of ordinary skill in the data processing arts to convey the substance of their work to others skilled in the art. An algorithm as described herein, and generally, is considered to be a self-consistent sequence of operations or similar processing leading to a desired result. In this context, operations or processing involve physical manipulation of physical quantities. Typically, although not necessarily, such quantities may take the form of electrical or magnetic signals capable of being stored, transferred, combined, compared or otherwise manipulated. It has been convenient at times, principally for reasons of common usage, to refer to such signals as bits, data, values, elements, symbols, characters, terms, numbers, numerals or the like. It should be understood, however, that all of these and similar terms are to be associated with appropriate physical quantities and are merely convenient labels. Unless specifically stated otherwise, as apparent from the following discussion, it is appreciated that throughout this specification discussions utilizing terms such as "processing," "computing," "calculating," "determining" or the like refer to actions or processes of a computing platform, such as a computer or a similar electronic computing device, that manipulates or transforms data represented as physical electronic or magnetic quantities within memories, registers, or other information storage devices, transmission devices, or display devices of the computing platform.

While this invention has been particularly shown and described with references to preferred examples thereof, it will be understood by those skilled in the art that various changes in form and details may be made therein without departing from the spirit and scope of the present application as defined by the appended claims. Such variations are intended to be covered by the scope of this present application. As such, the foregoing description of examples of the present application is not intended to be limiting. Rather, any limitations to the invention are presented in the following claims.

## Claims

1. An apparatus comprising:
an air elimination assembly including:
an input port to receive liquid;
an output port to output the liquid received through the input port;
a gas output port to expel gas from the liquid passing from the input port to the output port;
a first membrane disposed between the input port and the gas output port, the gas passing from the liquid through the first membrane to the gas output port; and
a pressure control source to control a magnitude of pressure at the gas output port to expel the gas from the liquid.

2. The apparatus as in claim 1, wherein the pressure control source is operative to set the magnitude of pressure at the gas output port to be less than a magnitude of pressure of the liquid inputted to the input port.

3. The apparatus as in claim 1, wherein the pressure control source is operative to control the magnitude of the pressure at the gas output port based on a surface tension of the liquid.

4. The apparatus as in claim 1, wherein the pressure control source is operative to:
monitor a magnitude of an input pressure of the liquid inputted to the input port;
derive a pressure value from the magnitude of the pressure of the liquid inputted to the input port; and
set the magnitude of the pressure at the gas output port to be the derived pressure value.

5. The apparatus as in claim 1 further comprising:
a diaphragm pump to force a flow of the liquid through the air elimination assembly to a recipient; and
wherein the pressure control source is operative to: i) apply positive pressure and
negative gas pressure to a chamber of the diaphragm pump; and ii) utilize the negative gas pressure to control to control the magnitude of pressure at the gas output port.

6. The apparatus as in claim 1 further comprising:
a second membrane disposed in a liquid flow path between the input port and the output port, the liquid passing through the second membrane.

7. The apparatus as in claim 6, wherein the first membrane is disposed at a higher position above a ground level in the air elimination assembly than the second membrane, a buoyancy of the gas causing at least a portion of the gas in the liquid to flow from a level of the second membrane to a level of the first membrane.

8. The apparatus as in claim 1 further comprising:
a second membrane through which the liquid passes from the input port to the output port; and
wherein the second membrane prevents passage of the gas to the output port.

9. The apparatus as in claim 1 further comprising:
a chamber disposed in the air elimination assembly between the input port and the output port, the chamber having a cross-section larger than a cross section of the input port, the larger cross-section of the chamber causing a flow velocity of the liquid through the chamber to be less than a flow velocity of the liquid through the input port.

10. The apparatus as in claim 1, wherein the air elimination assembly includes a chamber disposed between the input port and the output port; and
wherein the chamber includes a tapered pathway extending to the gas output port.

11. The apparatus as in claim 1, wherein the gas output port is disposed at a higher level than the input port with respect to a ground reference, a buoyancy of the gas causing at least a portion of the gas to flow towards the gas output port.

12. The apparatus as in claim 1 further comprising:
a pressure storage reservoir disposed in a fluid path between the pressure control source and the gas output port;
a valve disposed in the fluid path between the pressure control source and the pressure storage reservoir, the valve controlling a magnitude of gas pressure in the pressure storage reservoir; and
wherein the pressure storage reservoir is operative to apply the controlled gas pressure to the gas output port.

13. A method comprising:
receiving liquid at an input port of an air elimination assembly;
outputting the liquid received at the input port from an output port of the air elimination assembly;
expelling gas from a gas output port of the air elimination assembly, a first membrane disposed between the input port and the gas output port, the gas passing from the liquid through the first membrane to the gas output port; and
via a pressure control source, controlling a magnitude of pressure at the gas output port to expel the gas from the liquid.

14. The method as in claim 13 further comprising:
via the pressure control source, setting the magnitude of pressure at the gas output port to be less than a magnitude of pressure of the liquid inputted to the input port.

15. The method as in claim 13 further comprising:
via the pressure control source, controlling the magnitude of the pressure at the gas output port based on a surface tension of the liquid.
